# EUROPÄISCHE PATENTSCHRIFT

(11) **EP 3 898 744 B1**
(45) Veröffentlichungstag und Bekanntmachung des Hinweises auf die Patenterteilung: **21.08.2024**
(21) Anmeldenummer: 19832938.5
(22) Anmeldetag: 20.12.2019
(51) Int. Cl.: C08G 18/42, C08G 18/46, C07C 51/00, C08G 69/02, C08G 65/00, C08G 64/00, C08G 63/00, C08G 18/76, C08G 63/12, C08G 63/52, C08G 64/02, C08G 65/26, C08G 69/28, C07C 51/47

(54) **VERFAHREN ZUR HERSTELLUNG EINER MISCHUNG UMFASSEND MINDESTENS EINE VERBINDUNG MIT MINDESTENS ZWEI HYDROXY- UND/ODER AMINOGRUPPEN UND DEREN VERWENDUNG ZUR HERSTELLUNG EINES POLYMERS**
PROCESS FOR PREPARING A MIXTURE COMPRISING A COMPOUND HAVING AT LEAST TWO HYDROXYL- AND/OR AMINO GROUPS AND ITS USE FOR PREPARING A POLYMER
PROCÉDÉ DE PRÉPARATION D'UN MÉLANGE CONTENANT UN COMPOSÉ AYANT AU MOINS DEUX GROUPES HYDROXYL ET/OU AMINO ET SON UTILISATION POUR LA PRÉPARATION D'UN POLYMÈRE

(30) Priorität: 21.12.2018 DE 102018222887
(43) Veröffentlichungstag der Anmeldung: 27.10.2021
(73) Patentinhaber: RAMPF Advanced Polymers GmbH & Co. KG, 72661 Grafenberg (DE)
(72) Erfinder: KUGLER, Michael, 66954 Pirmasens (DE)
(74) Vertreter: Weickmann & Weickmann PartmbB
(86) Internationale Anmeldenummer: PCT/EP2019/086553
(87) Internationale Veröffentlichungsnummer: WO 2020/127890

(56) Entgegenhaltungen:
- EP-A1- 2 371 805
- DATABASE WPI Week 200253, Derwent World Patents Index; AN 2002-492739, XP002798331
- DATABASE WPI Week 201557, Derwent World Patents Index; AN 2015-47340X, XP002798332

## Beschreibung

Die vorliegende Erfindung betrifft ein Verfahren zur Herstellung einer Mischung umfassend mindestens eine Verbindung mit mindestens zwei Hydroxy- und/oder Aminogruppen, eine solche Mischung und deren Verwendung zur Herstellung eines Polymers, entsprechend der Ansprüche 1, 12 und 15.

Natürliche und insbesondere nachwachsende Rohstoffquellen werden in der heutigen Zeit immer wichtiger. Aus Aspekten der nachhaltigen Nutzung endlicher Ressourcen und aufgrund der Tatsache, dass fossile Rohstoffe nicht unbegrenzt zur Verfügung stehen, werden nachwachsende Rohstoffe immer relevanter.

Zur Herstellung von Kunststoffen werden große Mengen an Monomeren benötigt, die häufig aus fossilen Quellen stammen. Es ist daher seit vielen Jahren ein Ziel in der Kunststoffherstellung ein Loslösen von endlichen Rohstoffquellen zu erzielen und sich hin zu nachhaltigen, nachwachsenden Rohstoffen zu orientieren.

Zur Kunststoffherstellung werden Monomere benötigt, die funktionelle Gruppen tragen, um sie in einer Polymerisation zu Polymeren, d.h. langkettigen Molekülen umzusetzen. So werden für Polyadditionsreaktionen wie beispielsweise die Herstellung von Polyurethanen oder auch Polykondensationsreaktionen wie beispielsweise die Herstellung von Polyestern oder Polyamiden, Monomere mit mindestens zwei funktionellen Gruppen benötigt, die miteinander zur Reaktion gebracht werden.

So werden zum Beispiel Fettsäuren mit mehr als einer funktionellen Gruppe und Esterpolyole auf Basis von Fettsäuren mit mehr als einer funktionellen Gruppe, die aus natürlichen, d.h. nachwachsenden Rohstoffquellen stammen, bei der Herstellung von Polyolen bzw. Polyurethan-Kunststoffen erfolgreich eingesetzt. Ein Beispiel für die Verwendung einer derartigen Fettsäure aus nachwachsenden Quellen ist die Verwendung von Ricinolsäure als Monomer für die Herstellung von Esterpolyolen oder in Form des Glycerids (Rizinusöl) als Polyolkomponente bei der Polyurethanherstellung im industriellen Maßstab. Aufgrund ihrer zwei funktionellen Gruppen, nämlich der Hydroxygruppe und der Carboxygruppe, eignet sich die Ricinolsäure besonders gut als Monomer für die Polyol- und/oder Polyurethanherstellung.

Ebenso werden monofunktionelle Fettsäuren wie beispielsweise Ölsäure als Additiv unter anderem bei der Herstellung von aromatischen Esterpolyolen eingesetzt, um die Viskosität zu erniedrigen und hydrophobe Gruppen einzuführen. Der Nachteil solcher monofunktionellen Fettsäuren ist, dass diese bei der Polymerherstellung zum Kettenabbruch führen und deshalb nur in kleineren Konzentrationen zugesetzt werden können.

Eine Erhöhung der Funktionalität von ungesättigten Fettsäuren kann mittels Epoxydierung erreicht werden. Durch anschließende Ringöffnung, das heißt Hydroxylierung, können durch Umsetzung mit Wasser, Alkoholen und/oder Aminen entsprechende hochfunktionelle Polyole bzw. Polyamine hergestellt werden, die auch umfangreich für die Herstellung von Polyurethan-Kunststoffen eingesetzt werden.

Es gibt verschiedenste natürliche, nachwachsende Quellen, die mehrfunktionelle Fettsäuren enthalten, wie zum Beispiel Rinde. Rinde im Sinne der vorliegenden Erfindung umfasst insbesondere das sekundäre Abschlussgewebe (Periderm) und das tertiäre Abschlussgewebe (Borke) von Bäumen, stärker bevorzugt Kork und Borke. Hierbei sind insbesondere Quellen interessant, die in großen Mengen verfügbar sind und die bestenfalls als Nebenprodukt anfallen und somit einer neuen Verwendung zugeführt werden können, wie z.B. Birkenrinde. Birkenrinde fällt beispielsweise in großen Mengen als Nebenprodukt der Celluloseproduktion an und wird momentan energetisch verwertet, das heißt sie wird verbrannt.

Rinden enthalten in großen Mengen das natürliche Biopolymer Suberin, welches sich in vielen Zellwänden von Pflanzen findet. Nennenswerte Mengen von Suberin, welche sich typischerweise zwischen 30 und 60 Gew.% bewegen, sind in verschiedensten Pflanzenarten wie Kartoffeln und Baumwolle zu finden. Speziell finden sich jedoch in der Kutikula von Korkeiche, Douglastanne, Buche und Birke größere Mengen von Suberin. Ebenso wird auch Cutin gefunden, welches ein weiteres natürliches Biopolymer ist und ebenfalls in Pflanzen vorkommt.

Durch Extraktion können aus Rinde die darin enthaltenen Fettsäuren isoliert werden. Bei den aus Rinde extrahierten Fettsäuren handelt es sich um ein Gemisch umfassend Carbonsäuren, die mindestens eine weitere funktionelle Gruppe, z.B. eine Hydroxy-, Epoxy- und/oder Säuregruppe umfassen.

DE 829 447 C beschreibt die Gewinnung von höhermolekularen, organischen Säuren aus Rinden durch Extraktion.

In US 6,768,016 B2 wird ein Verfahren zur Gewinnung von Fettsäuren aus Birkenrinde und die Isolierung der einzelnen Fettsäuren beschrieben.

EP 2 371 805 A1 beschreibt die Herstellung von Oligo- und Polyestern aus Suberin- bzw. Cutin-Carbonsäuregemischen, die durch Hydrolyse gewonnen werden. Die nicht weiter getrennten Suberin-Fettsäuregemische werden mit Carbonsäuren verestert. Als Endprodukte entstehen Gemische aus Oligo-Carbonsäuren.

Für Polymerisationsreaktionen wie die Herstellung von Polyurethanen, Polyestern, Polyamiden und weiteren werden beispielsweise Polyole und Polyamine benötigt. Wenn entsprechende Substanzen aus natürlichen Quellen verwendet werden, müssen diese oftmals aufwendig extrahiert und insbesondere aufgereinigt werden, um für Polymerisationsreaktionen geeignet zu sein. Einige Verfahren schließen außerdem den Einsatz giftiger Substanzen wie Dimethylsulfat ein, deren Verwendung aus Gründen des Arbeitssicherheits- und Umweltschutzes minimiert werden sollte und zudem einen erhöhten Prozessaufwand verursacht.

Die oben genannten Aspekte sind Nachteile der bekannten Verfahren aus dem Stand der Technik. Es ist daher Aufgabe der vorliegenden Erfindung, die aus dem Stand der Technik bekannten Nachteile zu überwinden. Die vorliegende Erfindung ist auf ein Herstellungsverfahren, sowie auf Mischungen von Verbindungen und die Verwendung entsprechender Mischungen bei der Herstellung von Polymeren gerichtet, welche die oben genannten Nachteile überwinden.

Überraschenderweise hat sich gezeigt, dass nicht nur reine Fettsäuren, sondern auch die erfindungsgemäßen Mischungen zur Herstellung von Polymeren eingesetzt werden können. Aus den Mischungen können zum Beispiel Kunststoffe wie Polyurethane, Polyester oder Polyamide mit hervorragenden Produkteigenschaften hergestellt werden, die insbesondere in Bezug auf ihre mechanischen Eigenschaften wie beispielsweise Druck- und Biegefestigkeit mit Kunststoffen aus kommerziell erhältlichen, reinen Monomeren konkurrieren können bzw. diese sogar übertreffen. Zudem steigert die signifikante Vereinfachung des Verfahrens durch Vermeidung zusätzlicher Reinigungs- und Isolationsschritte, wie im Stand der Technik für gewöhnlich eingesetzt, die Wirtschaftlichkeit des Verfahrens und der daraus abgeleiteten Mischungen und Polymere durch Zeitersparnis, technische Aufwandsminimierung und Kostenreduktion in der Herstellung.

Weiterhin unterstützt die Verwendung der aus Biomasse-Material hergestellten erfindungsgemäßen Mischungen die Schonung von Rohstoffressourcen und erhöht den Anteil an nachwachsenden Rohstoffen im Kunststoffsektor. Ebenso wird ein Abfallprodukt durch Veredelung seiner Komponenten zu einem wertvollen Rohstoff für die weitere Wertschöpfung. Die Wahl eines leicht zugänglichen und günstigen Edukts steigert zudem die Wirtschaftlichkeit des erfindungsgemäßen Verfahrens.

Ein weiterer Vorteil bei der Verwendung von natürlichen Rohstoffquellen ist, dass die erhaltenen Substanzgemische eine Vielzahl an funktionellen Gruppen umfassen, die orthogonal funktionalisierbar sind und weitere maßgeschneiderte Funktionalisierungen für Spezialanwendungen zulassen.

### Darstellung der Erfindung

Die Erfindung betrifft ein Verfahren zur Herstellung einer Mischung umfassend mindestens eine Verbindung (I) mit mindestens zwei Hydroxy- und/oder Aminogruppen, wobei das Verfahren die folgenden Schritte umfasst:
a) Bereitstellen von mindestens einem Biomasse-Material, bevorzugt Schalen und/oder Rinde,
b) Behandeln des mindestens einen Biomasse-Materials aus Schritt a) mit mindestens einem ersten organischen Lösungsmittel bei erhöhter Temperatur, nämlich >20 °C, bevorzugt 30-200 °C,
c) Abtrennen des Feststoffs aus der nach Schritt b) erhaltenen Mischung,
d) Behandeln des nach Schritt c) erhaltenen Feststoffs mit mindestens einem zweiten Lösungsmittel unter alkalischen Bedingungen bei erhöhter Temperatur, nämlich >20 °C, bevorzugt bei 30-200 °C,
e) Abtrennen der flüssigen Phase aus der nach Schritt d) erhaltenen Mischung,
f) zumindest teilweises Entfernen des mindestens einen zweiten Lösungsmittels aus der nach Schritt e) erhaltenen flüssigen Phase,
g) Umsetzen der nach Schritt f) erhaltenen Mischung mit mindestens einer Verbindung (II) mit mindestens zwei Hydroxy- und/oder Aminogruppen.

In Schritt a) wird mindestens ein Biomasse-Material, das heißt ein Biomasse-Material oder eine Mischung von zwei oder mehreren Biomasse-Materialien, bereitgestellt. Das Biomasse-Material kann biologisches Material umfassen, beispielsweise Pflanzen oder von Pflanzen abgeleitetes Material, wie Folgeprodukte, Nebenprodukte, Rückstände oder Abfälle. Das Biomasse-Material kann als Frischbiomasse, d.h. Biomasse einschließlich zumindest eines Teils des natürlich enthaltenen Wassers, als Trockenbiomasse, oder als Mischung davon vorliegen. Ggf. stammt das Biomasse-Material von Bäumen und/oder Sträuchern. Biomasse-Material von Bäumen kann beispielsweise von Birken, Buchen, Eichen, bevorzugt Korkeichen, Kiefern, Tannen, bevorzugt Douglastannen, und/oder Fichten stammen. Biomasse-Material von Sträuchern kann beispielsweise von Eiben, Flieder und/oder Ginster stammen. Das Biomasse-Material kann von unterirdischen oder überirdischen Pflanzenorganen stammen und beispielsweise Wurzeln, Fruchtschalen, Samen, Samenhaare, wie z.B. Baumwolle, und/oder Rinde umfassen. Fruchtschalen umfassen beispielsweise Kartoffelschalen und Maisschalen. In einer Ausführungsform umfasst das Biomasse-Material Rinde, insbesondere das sekundäre und/oder tertiäre Abschlussgewebe von Bäumen, stärker bevorzugt Kork und/oder Borke, insbesondere der Birke.

Das Biomasse-Material kann unbehandelt oder in zerkleinerter Form bereitgestellt werden. In einer Ausführungsform wird das Biomasse-Material in zerkleinerter Form bereitgestellt, beispielsweise als Schnittgut, Schreddergut, Pellet, Schnitzel, Span, Granulat, Faser, Mahlgut, Pulver oder als Mischung davon. Zerkleinertes Biomasse-Material hat typischerweise eine Teilchengröße von ungefähr 0,1 bis 10 cm, bevorzugt bis 5 cm, stärker bevorzugt bis 2 cm und noch stärker bevorzugt bis 1 cm.

In einer Ausführungsform wird das mindestens eine Biomasse-Material in Schritt b) mit einem ersten organischen Lösungsmittel behandelt. In einer Ausführungsform wird das mindestens eine Biomasse-Material in Schritt b) mit mehr als einem ersten organischen Lösungsmittel, d.h. mit einem Lösungsmittelgemisch, wie beispielsweise einem Gemisch aus zwei, drei oder vier Lösungsmitteln, behandelt. Bevorzugt umfasst das mindestens eine erste organische Lösungsmittel Aceton, Dichlormethan, Benzol oder einer Mischung davon. Bevorzugt umfasst das mindestens eine erste organische Lösungsmittel Aceton.

Schritt b) wird bei einer erhöhten Temperatur, d.h. > 20 °C, durchgeführt. Bevorzugt wird Schritt b) bei 30-200 °C, stärker bevorzugt bei 50-150 °C, noch stärker bevorzugt bei 60-120 °C durchgeführt.

In einer Ausführungsform wird Schritt b) bei Normaldruck, d.h. bei ungefähr 1 bar, durchgeführt. In einer anderen Ausführungsform wird Schritt b) bei vermindertem Druck, d.h. bei weniger als 1 bar, durchgeführt. Bevorzugt wird Schritt b) bei vermindertem Druck wie beispielsweise bei 150-800 mbar, bevorzugt 150-500 mbar, stärker bevorzugt 200-400 mbar durchgeführt.

Schritt b) wird beispielsweise für 1-300 h, bevorzugt 20-400 h, noch stärker bevorzugt 60-300 h und noch stärker bevorzugt 90-180 h durchgeführt. Die Dauer des Verfahrensschritts b) kann beispielsweise von der Art, Menge und Teilchengröße des mindestens einen Biomasse-Materials; der Art und Menge des mindestens einen ersten organischen Lösungsmittels; und der Art und Menge der zu extrahierenden Substanz abhängen. Die zu extrahierende Substanz kann beispielsweise eine unpolare, organische Substanz sein und ggf. Betulin, Betulinsäure, Lupeol und/oder Derivate davon umfassen. Die zu extrahierende Substanz liegt bevorzugt gelöst in dem ersten organischen Lösungsmittel vor.

Abtrennen des Feststoffs in Schritt c) kann beispielsweise Filtration wie Heißfiltration, Zentrifugation, und/oder Dekantation umfassen. Schritt c) kann ferner auch das Waschen des abgetrennten Feststoffs mit mindestens einem ersten Lösungsmittel und anschließende Filtration umfassen.

In einer Ausführungsform wird der nach Schritt c) erhaltene Feststoff in Schritt d) mit einem zweiten Lösungsmittel behandelt. In einer Ausführungsform wird der nach Schritt c) erhaltene Feststoff in Schritt d) mit mehr als einem zweiten Lösungsmittel, d.h. mit einem Lösungsmittelgemisch, behandelt. Das Lösungsmittelgemisch umfasst mindestens zwei, d.h. zwei, drei, vier oder mehr, Lösungsmittel. Bevorzugt umfasst das mindestens eine zweite Lösungsmittel mindestens ein protisches Lösungsmittel. Das mindestens eine zweite Lösungsmittel ist bevorzugt Isopropanol, Ethanol, Wasser oder einer Mischung davon. Bevorzugt umfasst das mindestens eine zweite Lösungsmittel Isopropanol.

Schritt d) wird bevorzugt in Gegenwart mindestens einer alkalischen Verbindung, wie beispielsweise in Gegenwart eines Hydroxids, eines Carbonats, eines Hydrogencarbonats oder Mischungen davon durchgeführt. Stärker bevorzugt wird Schritt d) in Gegenwart eines Hydroxids wie NaOH und/oder KOH, insbesondere NaOH durchgeführt. Die mindestens eine alkalische Verbindung ist bevorzugt in einem des mindestens einen zweiten Lösungsmittels gelöst. Die mindestens eine alkalische Verbindung ist in der Lage in Schritt d) alkalische Bedingungen, d.h. einen pH-Wert > 7,0, zu erzeugen. In einer Ausführungsform beträgt der pH-Wert in Schritt d) > 7,0 -14,0 und bevorzugt > 8,0 - 14,0.

Schritt d) wird bei einer erhöhten Temperatur d.h. > 20 °C, durchgeführt. Bevorzugt wird Schritt d) bei 30-200 °C, stärker bevorzugt bei 50-150 °C, noch stärker bevorzugt bei 60-120 °C durchgeführt.

In einer Ausführungsform wird Schritt d) bei Normaldruck, d.h. bei ungefähr 1 bar, durchgeführt. In einer anderen Ausführungsform wird Schritt d) bei vermindertem Druck, d.h. bei weniger als 1 bar, durchgeführt.

Die Dauer des Verfahrensschritts d) kann beispielsweise von dem mindestens einen Biomasse-Material, der Teilchengröße des mindestens einen Biomasse-Materials, dem mindestens einen zweiten Lösungsmittel und dem Massenverhältnis von Biomasse-Material zu Lösungsmittel abhängen. Schritt d) wird beispielsweise für 1-1000 min, stärker bevorzugt 20-500 min, noch stärker bevorzugt 60-300 min, insbesondere 90-180 min durchgeführt.

In Schritt d) werden bevorzugt Fettsäuren und/oder Derivate davon aus dem Feststoff in die Lösungsmittelphase extrahiert. Fettsäuren und Derivate davon im Sinne der Erfindung sind Verbindungen, die wenigstens eine Carboxylgruppe, bevorzugt eine oder zwei Carboxylgruppen, und einen verzweigten oder unverzweigten, gesättigten oder ungesättigten Kohlenwasserstoffrest, bevorzugt mit 12-26 Kohlenstoffatomen, umfassen. Fettsäuren und Derivate davon im Sinne der Erfindung können mindestens eine weitere funktionelle Gruppe aufweisen wie zum Beispiel mindestens eine Hydroxygruppe, mindestens eine Epoxygruppe und/oder mindestens eine ungesättigte C-C-Bindung. Insbesondere werden in Schritt d) Fettsäuren und Derivate davon umfassend gesättigte und/oder ungesättigte C₁₂-C₂₆-Fettsäuren und Derivate davon, bevorzugt gesättigte und/oder ungesättigte C₁₂-C₂₄-Fettsäuren und Derivate davon, bevorzugt gesättigte und/oder ungesättigte C₁₄-C₂₄-Fettsäuren und Derivate davon und stärker bevorzugt gesättigte und/oder ungesättigte C₁₄-C₂₂-Fettsäuren und Derivate davon extrahiert. Bevorzugt werden Fettsäuren und Derivate davon umfassend Octadec-9-endisäure, 18-Hydroxyoctadec-9-ensäure, 9,16-Dihydroxyhexadecansäure, 9,10-Epoxy-18-hydroxyoctadecansäure, 20-Hydroxyeicosansäure, 9,10,18-Trihydroxyoctadecansäure, Docosandisäure, 22-Hydroxydocosansäure oder Mischungen davon extrahiert.

Bevorzugt wird Schritt d) unter Bedingungen durchgeführt, bei denen die in den Fettsäuren und/oder Derivaten davon enthaltenen weiteren funktionellen Gruppen unverändert bleiben, d.h. nicht reagieren. Dies wird insbesondere durch die erfindungsgemäßen milden Reaktionsbedingungen gewährleistet. Bevorzugt werden keine starken Säuren oder Basen bei dem Extraktionsschritt verwendet, um beispielsweise einer Ringöffnung von ggf. enthaltenen Epoxiden vorzubeugen.

Abtrennen der flüssigen Phase in Schritt e) kann beispielsweise Filtration, Zentrifugation und/oder Dekantation umfassen. Bevorzugt umfasst Schritt e) Heißfiltration der flüssigen Phase. Die Temperatur bei der Heißfiltration ist > 20 °C und ist bevorzugt 30-200 °C, stärker bevorzugt 50-150 °C und noch stärker bevorzugt 60-120 °C. Die zu extrahierenden Fettsäuren und/oder Derivate davon befinden sich bevorzugt in dem mindestens einen zweiten Lösungsmittel. Schritt e) kann ferner das Waschen des abgetrennten Feststoffs mit mindestens einem zweiten Lösungsmittel und anschließende Filtration umfassen. Im letzteren Fall werden die flüssigen Phasen vereint.

Zumindest teilweises Entfernen des mindestens einen zweiten Lösungsmittels in Schritt f) kann beispielsweise Destillation, Filtration, Eindampfen, Zentrifugation und/oder Vakuumtrocknen umfassen. Bevorzugt umfasst Schritt f) Abkühlen der nach Schritt e) erhaltenen flüssigen Phase, bevorzugt auf eine Temperatur ≤ 20 °C, insbesondere ≤ 0 °C, und anschließende Filtration. Unter zumindest teilweisem Entfernen in Schritt f) wird verstanden, dass mindestens 70 Gew.-% des mindestens einen zweiten Lösungsmittels bezogen auf die Gesamtmasse des mindestens einen zweiten Lösungsmittels entfernt werden. Bevorzugt werden mindestens 80 Gew.%, stärker bevorzugt mindestens 90 Gew.-%, noch stärker bevorzugt mindestens 95 Gew.-%, und noch stärker bevorzugt mindestens 99 Gew.-% des mindestens einen zweiten Lösungsmittels bezogen auf die Gesamtmasse des mindestens einen zweiten Lösungsmittels entfernt. In einer bevorzugten Ausführungsform wird das mindestens eine zweite Lösungsmittel so weit entfernt, bis sich bei Raumtemperatur, d.h. bei 20 °C, ein fester Rückstand bildet.

Ggf. kann die in Schritt f) erhaltene Mischung vor dem Umsetzen in Schritt g) mindestens einem Reinigungsschritt, d.h. einem, zwei, drei oder mehr Reinigungsschritten, unterzogen werden. Ein geeigneter Reinigungsschritt kann beispielsweise Umkristallisation, Resuspendieren in einem polaren Lösungsmittel bzw. Lösungsmittelgemisch, wie Wasser und/oder ein Alkohol, und anschließendes zumindest teilweises Entfernen des polaren Lösungsmittels bzw. Lösungsmittelgemischs, oder andere dem Fachmann bekannte Verfahren umfassen.

Die in Schritt f) erhaltene Mischung umfassend Fettsäuren und/oder Derivate davon kann wie oben beschrieben auch Epoxygruppen und/oder ungesättigte C-C Bindungen enthalten.

Im Schritt g) wird die nach Schritt f) erhaltene Mischung mit mindestens einer Verbindung (II) mit mindestens zwei Hydroxy- und/oder Aminogruppen umgesetzt. Verbindung (II) kann also entweder
- mindestens zwei Hydroxygruppen, bevorzugt 2-5 Hydroxygruppen, oder
- mindestens zwei Aminogruppen, bevorzugt 2-5 Aminogruppen, oder
- mindestens eine Hydroxy- und mindestens eine Aminogruppe, bevorzugt eine Hydroxy- und eine Aminogruppe,
umfassen.

Verbindung (II) ist bevorzugt ausgewählt aus der Gruppe bestehend aus einem Polyol wie beispielsweise einem Diol, Triol, Tetraol oder Pentaol, einem Aminopolyol wie beispielsweise einem Aminodiol, Aminotriol, Aminotetraol oder Aminopentaol, einem Polyamin wie beispielsweise einem Diamin, Triamin, Tetraamin oder Pentaamin, einem Polyaminoalkohol wie beispielsweise einem Diaminmonoalkohol, Triaminmonoalkohol, Tetraaminmonoalkohol oder Pentaaminmonoalkohol, einem Aminoalkohol d.h. einem Monoaminomonoalkohol, einem Polyaminopolyol, oder einer Mischung davon.

Bevorzugt ist Verbindung (II) ausgewählt aus linearen, cyclischen oder verzweigten Polyalkylenoxid-, Alkyl- und Alkenylverbindungen mit mindestens zwei Hydroxy- und/oder Aminogruppen, und Mischungen davon. Stärker bevorzugt ist Verbindung (II) ausgewählt aus linearen, cyclischen oder verzweigten Poly(C₂-C₅-Alkylenoxid)-, C₁-C₃₀-Alkyl- und C₂-C₃₀-Alkenylverbindungen mit mindestens zwei Hydroxy- und/oder Aminogruppen, und Mischungen davon. Noch stärker bevorzugt ist Verbindung (II) ausgewählt aus linearen, cyclischen oder verzweigten Poly(C₂-C₅-Alkylenoxid)-, C₁-C₁₀-Alkyl- und C₂-C₁₀-Alkenylverbindungen mit mindestens zwei Hydroxy- und/oder Aminogruppen, und Mischungen davon. Insbesondere ist Verbindung (II) ausgewählt aus einem Diol, Triol, Diamin, Triamin, Aminoalkohol oder einer Mischung davon, bevorzugt aus Diethylenglykol, Dipropylenglykol, Ethanolamin, Ethylendiamin, Propandiamin, Butandiol, 1,2-Butandiamin, 1,3-Butandiamin oder einer Mischung davon, insbesondere Diethylenglykol.

Umsetzen der nach Schritt f) erhaltenen Mischung in Schritt g) mit mindestens einer Verbindung (II) erfolgt bei erhöhter Temperatur, d.h. bei > 20 °C. Bevorzugt beträgt die Temperatur in Schritt g) 50-400 °C, stärker bevorzugt 150-300 °C, noch stärker bevorzugt 200-300 °C.

In einer Ausführungsform wird Schritt g) bei Normaldruck, d.h. bei ungefähr 1 bar, durchgeführt. In einer anderen Ausführungsform wird Schritt g) bei vermindertem Druck, d.h. bei weniger als 1 bar, durchgeführt. Bevorzugt wird Schritt g) bei vermindertem Druck wie beispielsweise 150-800 mbar, bevorzugt 150-500 mbar, stärker bevorzugt 150-350 mbar durchgeführt.

Bevorzugt wird Schritt g) in Gegenwart eines Katalysators durchgeführt. In einer Ausführungsform wird das Umsetzen in Schritt g) ohne Katalysator durchgeführt. In einer Ausführungsform umfasst Schritt g) Destillation und bevorzugt AzeotropDestillation. In einer bevorzugten Ausführungsform wird das Umsetzen in Schritt g) in Gegenwart eines Katalysators durchgeführt. Geeignete Katalysatoren sind beispielsweise metallorganische und/oder basische Katalysatoren. Beispiele für geeignete Katalysatoren umfassen Titantetrabutanolat, Zinnoctoat, Manganacetat, Dibutylzinndilaurat, Kaliumacetat, Kaliumhydroxid oder eine Kombination davon, bevorzugt Titantetrabutanolat.

Die Dauer des Schritts g) ist beispielsweise von der Art und Konzentration der mindestens einen Verbindung (II), der Reaktionstemperatur, dem Reaktionsdruck und ggf. der Art und Konzentration des Katalysators abhängig. Schritt g) wird beispielsweise für 30-2400 min, stärker bevorzugt 120-1200 min, noch stärker bevorzugt 180-600 min durchgeführt.

Ferner betrifft die Erfindung ein Verfahren zur Herstellung einer Mischung umfassend mindestens eine Verbindung (I) mit mindestens zwei Hydroxy- und/oder Aminogruppen, wobei das Verfahren die folgenden Schritte umfasst:
a) Bereitstellen von mindestens einem Biomasse-Material, bevorzugt Schalen und/oder Rinde,
b) Behandeln des mindestens einen Biomasse-Materials aus Schritt a) mit mindestens einem ersten organischen Lösungsmittel bei erhöhter Temperatur, nämlich >20 °C,
c) Abtrennen des Feststoffs aus der nach Schritt b) erhaltenen Mischung,
d) Behandeln des nach Schritt c) erhaltenen Feststoffs mit mindestens einem zweiten Lösungsmittel unter alkalischen Bedingungen bei erhöhter Temperatur, nämlich >20 °C,
e) Abtrennen der flüssigen Phase aus der nach Schritt d) erhaltenen Mischung,
f) zumindest teilweises Entfernen des mindestens einen zweiten Lösungsmittels aus der nach Schritt e) erhaltenen flüssigen Phase,
   f1) zumindest teilweises Umsetzen der nach Schritt f) erhaltenen Mischung, enthaltend Epoxygruppen und/oder ungesättigte C-C Bindungen, in einer Oxidations- und/oder Additionsreaktion, und
g) Umsetzen der nach Schritt f1) erhaltenen Mischung mit mindestens einer Verbindung (II) mit mindestens zwei Hydroxy- und/oder Aminogruppen.

Obenstehende Spezifikationen bezüglich der Schritte a), b), c), d), e), und f) sind auf das erfindungsgemäße Verfahren umfassend den zusätzlichen Schritt f1) anzuwenden.

Zumindest teilweises Umsetzen in Schritt f1) kann eine Oxidationsreaktion und/oder eine Additionsreaktion umfassen. In einer Ausführungsform umfasst das zumindest teilweise Umsetzen in Schritt f1) eine Oxidationsreaktion und bevorzugt Ozonolyse oder Wasserstoffperoxid-vermittelte Oxidation. In einer Ausführungsform umfasst das zumindest teilweise Umsetzen in Schritt f1) eine Additionsreaktion und bevorzugt eine nukleophile Addition oder eine Cycloaddition, wie beispielsweise eine Diels-Alder-Reaktion. Die Additionsreaktion kann Umsetzen mit einem ungesättigten Säureanhydrid, bevorzugt Maleinsäureanhydrid, einem Mono- oder Polyamin, einem Mono- oder Polyol, oder einer Mischung davon umfassen.

Zumindest teilweises Umsetzten in Schritt f1) bedeutet ein Umsetzten von mindestens 50 % der für die Umsetzungsreaktion reaktiven funktionellen Gruppen der nach Schritt f) erhaltenen Mischung. Bevorzugt bedeutet zumindest teilweises Umsetzten ein Umsetzten von 50-100 %, bevorzugt 60-100%, stärker bevorzugt 70-100%, noch stärker bevorzugt 80-100% und noch stärker bevorzugt 90-100% der für die Umsetzungsreaktion reaktiven funktionellen Gruppen der nach Schritt f) erhaltenen Mischung. Beispielshafte reaktive funktionelle Gruppen der nach Schritt f) erhaltenen Mischung sind Epoxygruppen, Hydroxygruppen, Carboxygruppen, ungesättigte C-C-Bindungen, beispielsweise C-C-Doppelbindungen, und/oder Estergruppen.

Zumindest teilweises Umsetzen in Schritt f1) kann ggf. bei erhöhter Temperatur, d.h. bei > 20 °C, beispielsweise bei 20-200 °C durchgeführt werden.

Ggf. wird Schritt f1) in Gegenwart eines Katalysators durchgeführt. In einer Ausführungsform wird das Umsetzen in Schritt f1) ohne Katalysator durchgeführt. In einer anderen Ausführungsform wird das Umsetzen in Schritt f1) in Gegenwart eines Katalysators durchgeführt. Geeignete Katalysatoren sind beispielsweise metallorganische und/oder basische Katalysatoren. Beispiele für geeignete Katalysatoren umfassen Titantetrabutanolat, Zinnoctoat, Manganacetat, Dibutylzinndilaurat, Kaliumacetat, Kaliumhydroxid oder eine Kombination davon, bevorzugt Titantetrabutanolat.

Obige Ausführungen bezüglich des Schrittes g) können analog auf das Umsetzen der nach Schritt f1) erhaltenen Mischung angewendet werden.

Durch das erfindungsgemäße Umsetzen in Schritt g) und ggf. Schritt f1) werden Mischungen erhalten, die sich durch eine gesteigerte Funktionalität auszeichnen. Im Vergleich zu Verbindungen aus dem Stand der Technik ist die Anzahl an Hydroxy- und/oder Aminogruppen in der Mischung erhöht. Dies ist insbesondere bezüglich der Reaktivität der Mischung, beispielsweise in einer Polymerisationsreaktion, vorteilhaft. Überraschenderweise wurde gefunden, dass aus einer Polymerisation der Mischung resultierende Polymere verbesserte mechanische Eigenschaften aufweisen. Zudem folgt aus dem erfindungsgemäßen Umsetzen in Schritt g) und ggf. Schritt f1) eine Mischung mit verbesserter Viskosität, die sich ohne weitere Zwischenschritte zur Herstellung von Polymeren wie beispielsweise Polyurethanen oder Polyestern eignet. Ferner weisen die erfindungsgemäßen Mischungen eine verbesserte Emulgierbarkeit mit hydrophoben Treibmitteln wie z.B. Pentan auf, was sich insbesondere auf die Herstellung von Polymerschäumen vorteilhaft auswirkt.

Ferner betrifft die Erfindung eine Mischung umfassend mindestens eine Verbindung (I) mit mindestens zwei Hydroxy- und/oder Aminogruppen, erhältlich durch ein oben beschriebenes Verfahren. Verbindung (I) umfasst bevorzugt mindestens eine Verbindung A, B, C und/oder D mit jeweils mindestens zwei Hydroxy- und/oder Aminogruppen, wobei Verbindung A 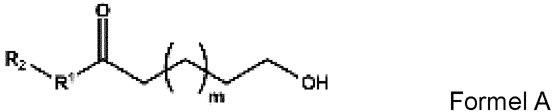 ist, mit m = 8-22, bevorzugt 10-22, stärker bevorzugt 12-22, noch stärker bevorzugt 12-20 und noch stärker bevorzugt 14-18,
wobei Verbindung B 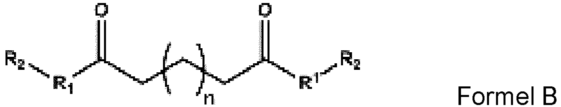 ist, mit n = 8-22, bevorzugt 10-22, stärker bevorzugt 12-22, noch stärker bevorzugt 12-20 und noch stärker bevorzugt 14-18,
wobei Verbindung C 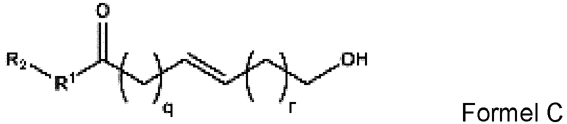 ist, mit q = 4 - 11 und r = 4 - 11, bevorzugt q = 5 - 11 und r = 5 - 11, stärker bevorzugt q = 6 - 11 und r = 6 - 11, noch stärker bevorzugt q = 6 - 10 und r = 6 - 10 und noch stärker bevorzugt q = 7 - 9 und r = 7 - 9,
und wobei Verbindung D 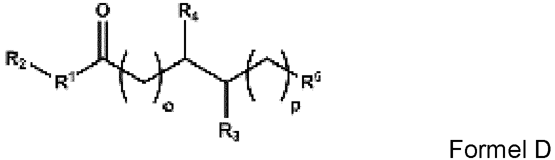 ist, mit o = 4 - 11 und p = 4 - 11, bevorzugt o = 5 - 11 und p = 5 - 11, stärker bevorzugt o = 6 - 11 und p = 6 - 11, noch stärker bevorzugt o = 6 - 10 und p = 6 - 10 und noch stärker bevorzugt o = 7 - 9 und p = 7 - 9,
wobei
R¹ = O oder NH ist;
R² ausgewählt ist aus der Gruppe bestehend aus einem Monoalkohol, Polyol, Monoamin, Polyamin, Polyaminoalkohol, Aminoalkohol, Aminopolyol, Polyaminopolyol oder einer Mischung davon;
R³ ausgewählt ist aus OH, O-R² oder NH-R²;
R⁴ ausgewählt ist aus OH, O-R² oder NH-R²;
Mit der Maßgabe, dass R³ = OH ist, wenn R⁴ = O-R² oder NH-R² ist und mit der Maßgabe, dass R⁴ = OH ist, wenn R³ = O-R² oder NH-R² ist; und
R⁵ ausgewählt ist aus -COR¹R² und -CH₂OH.

Bevorzugt ist R² ausgewählt aus linearen, cyclischen oder verzweigten Polyalkylenoxid-, Alkyl- und Alkenylverbindungen mit mindestens einer Hydroxy- und/oder Aminogruppen, und Mischungen davon. Mindestens eine Hydroxy- und/oder Aminogruppen umfasst mindestens eine Hydroxygruppen; mindestens eine Aminogruppen; und mindestens eine Hydroxygruppe und mindestens eine Aminogruppe.

Stärker bevorzugt ist R² ausgewählt aus linearen, cyclischen oder verzweigten Poly(C₂-C₅-Alkylenoxid)-, C₁-C₃₀-Alkyl- und C₂-C₃₀-Alkenylverbindungen mit mindestens einer Hydroxy- und/oder Aminogruppen, und Mischungen davon. Noch stärker bevorzugt ist R² ausgewählt aus linearen, cyclischen und verzweigten Poly(C₂-C₅-Alkylenoxid)-, C₁-C₁₀-Alkyl- und C₂-C₁₀-Alkenylverbindungen mit mindestens einer Hydroxy- und/oder Aminogruppen, und Mischungen davon. Insbesondere ist R² ausgewählt aus einem Monoalkohol, einem Diol, einem Monoamin, einem Diamin und einem Aminoalkohol einer linearen, cyclischen oder verzweigten Poly(C₂-C₅-Alkylenoxid)-, C₁-C₁₀-Alkyl- oder C₂-C₁₀-Alkenylverbindung oder einer Mischung davon.

Insbesondere bevorzugt ist R² ausgewählt aus CH₂CH₂OCH₂CH₃, einem Dipropylenglykol-Rest wie CH₂CH(CH₃)OCH(CH₃)CH₂OH, CH(CH₃)CH₂OCH₂CH(OH)CH₃, oder CH₂CH(CH₃)OCH₂CH(OH)CH₃, CH₂CH₂OH, CH₂CH₂NH₂, einem Propanamin-Rest wie CH₂CH₂CH₂NH₂ oder CH₂CH(NH₂)CH₃, einem Butanol-Rest wie CH₂CH₂CH₂CH₂OH, CH₂CH(OH)CH₂CH₃, oder CH₂CH₂CH(OH)CH₃, einem Butanamin-Rest wie CH₂CH₂CH₂CH₂NH₂, CH₂CH(NH₂)CH₂CH₃, oder CH₂CH₂CH(NH₂)CH₃ oder einer Mischung davon, insbesondere CH₂CH₂OCH₂CH₃.

Die Mischung umfassend mindestens eine Verbindung (I) mit mindestens zwei Hydroxy- und/oder Aminogruppen weist bevorzugt eine Viskosität (bestimmt nach dem Verfahren gemäß DIN 53019) von 1-250.000 mPas/50°C, bevorzugt 1-100.000 mPas/50°C, stärker bevorzugt 1-10.000 mPas/50°C und noch stärker bevorzugt 5-10.000 mPas/50°C auf.

Die Mischung umfassend mindestens eine Verbindung (I) mit mindestens zwei Hydroxy- und/oder Aminogruppen weist ggf. eine Hydroxylzahl (bestimmt nach dem Verfahren gemäß ASTM E 1899-08) von 1-1000 mg KOH/g, bevorzugt 5-800 mg KOH/g, stärker bevorzugt 50-700 mg KOH/g auf.

Der Anteil der Verbindungen A, B, C und/oder D an der Gesamtmischung beträgt bevorzugt mindestens 20 Gew.%, stärker bevorzugt mindestens 30 Gew.%, noch stärker bevorzugt mindestens 40 Gew.% und am stärksten bevorzugt mindestens 50 Gew.%.

Beim Umsetzen in Schritt g) können auch Verbindungen aus der nach Schritt f) bzw. f1) erhaltenen Mischung miteinander reagieren. Die Mischung umfassend mindestens eine Verbindung (I) mit mindestens zwei Hydroxy- und/oder Aminogruppen kann somit ggf. Homo- und/oder Heterodimere, Homo- und/oder Heterotrimere, Homo- und/oder Heterooligomere und/oder Homo- und/oder Heteropolymere von Verbindungen aus der nach Schritt f) bzw. f1) erhaltenen Mischung umfassen. Homodimere, -trimere, -oligomere und -polymere im Sinne der Erfindung sind Moleküle, die aus gleichartigen Bausteinen aufgebaut sind. Heterodimere, -trimere, -oligomere und -polymere im Sinne der Erfindung sind Moleküle, die aus unterschiedlichen Bausteinen aufgebaut sind. Die so erhaltenen Substanzen können ebenfalls mindestens zwei Hydroxy- und/oder Aminogruppen umfassen.

Bevorzugt umfasst die Mischung umfassend mindestens eine Verbindung (I) mit mindestens zwei Hydroxy- und/oder Aminogruppen Homo- und/oder Heterodimere, Homo- und/oder Heterotrimere, Homo- und/oder Heterooligomere und/oder Homo- und/oder Heteropolymere, die durch die Umsetzung der Substanzen A, B, C und/oder D miteinander entstehen. Stärker bevorzugt umfasst die Mischung umfassend mindestens eine Verbindung (I) mit mindestens zwei Hydroxy- und/oder Aminogruppen Homo- und/oder Heterodimere, Homo- und/oder Heterotrimere und/oder Homo- und/oder Heterooligomere, die durch die Umsetzung der Substanzen A, B, C und/oder D miteinander entstehen. Noch stärker bevorzugt umfasst die Mischung umfassend mindestens eine Verbindung (I) mit mindestens zwei Hydroxy- und/oder Aminogruppen Homo- und/oder Heterodimere und/oder Homo- und/oder Heterotrimere, die durch die Umsetzung der Substanzen A, B, C und/oder D miteinander entstehen.

Ein Aspekt der Erfindung betrifft die Verwendung der Mischung umfassend mindestens eine Verbindung (I) mit mindestens zwei Hydroxy- und/oder Aminogruppen zur Herstellung eines Polymers, wobei das Polymer ggf. ein Polyurethan, Polyisocyanurat, Polyharnstoff, Polyester, Polyamid, Polycarbonat, Polyether oder eine Kombination hieraus ist. Bevorzugt wird die Mischung umfassend mindestens eine Verbindung (I) mit mindestens zwei Hydroxy- und/oder Aminogruppen zur Herstellung eines Polyurethans verwendet. Das Polymer kann beispielsweise als Feststoff, z.B. als Pulver, Schaum, Plättchen oder Folie, vorliegen. Bevorzugt wird die Mischung umfassend mindestens eine Verbindung (I) mit mindestens zwei Hydroxy- und/oder Aminogruppen zur Herstellung eines Polymerschaums und stärker bevorzugt eines Polyurethanschaums verwendet.

Ein Aspekt der Erfindung betrifft ferner ein Polyurethan, umfassend eine Mischung umfassend mindestens eine Verbindung (I) mit mindestens zwei Hydroxy- und/oder Aminogruppen als ein Polymerbaustein.

### Herstellungsbeispiele

### Bestimmung der Viskosität

Die Viskosität wurde gemäß DIN 53019 unter Verwendung eines DV-II Viskosimeters mit einer LV 64 Spindel (Fa. Brookfield) bestimmt.

### Bestimmung der Hydroxylzahl

Die Hydroxylzahl wurde gemäß ASTM E 1899-08 unter Verwendung einer 907 Titrando Messvorrichtung (Fa. Metrohm) bestimmt.

### Ermittlung der Druckfestigkeit

Die Druckfestigkeit wurde gemäß DIN 53421 unter Verwendung einer Universalprüfmaschine Z010 (Fa. Zwick Roell) durchgeführt.

### Ermittlung der Biegefestigkeit

Die Biegefestigkeit wurde nach DIN EN ISO 178 unter Verwendung einer Universalprüfmaschine Z010 (Fa. Zwick Roell) durchgeführt.

### Verwendete Substanzen

Diethylenglykol wurde von der Firma Brenntag GmbH (Essen, Deutschland) bezogen.

Das Isocyanat mit dem Handelsnamen Desmodur 44V20 wurde von der Firma Covestro bezogen.

Tetrabutyltitanat wurde von der Firma Lehmann & Voss & Co. KG (Hamburg, Deutschland) unter dem Handelsnamen Tyzor TnBT bezogen.

Das verwendete Referenzpolyol wurde von der Firma Covestro unter der Handelsbezeichnung Baymer VP.PU 25HB55 bezogen.

Der Stabilisator mit der Handelsbezeichnung DC 193 wurde von der Firma Evonik (Deutschland) bezogen. Der Stabilisator mit der Bezeichnung LV 33 wurde ebenfalls von der Firma Evonik (Deutschland) bezogen.

Der Katalysator PC CAT NP10 (NP 10) wurde von der Firma Nitroil Europe Handels GmbH (Deutschland) bezogen.

### Herstellung Polyesterpolyol

### Beispiel 1

Birkenrinde wurde nach einem aus dem Stand der Technik bekannten Verfahren umgesetzt, welches Behandeln der Birkenrinde in Gegenwart von Aceton mehrere Stunden bei erhöhter Temperatur und Refluxieren in Gegenwart von Isopropanol und NaOH umfasst, wobei die in der Birkenrinde enthaltenen Fettsäuren dabei in das Lösungsmittel übergehen. Nach Abtrennung des Lösungsmittels und Aufreinigung und Trocknung wurde ein bei Raumtemperatur festes Fettsäuregemisch gewonnen. Dieses Fettsäuregemisch setzte sich aus verschiedenen, mehrfunktionellen Fettsäuren zusammen, die sowohl freie Säurefunktionen als auch Esterfunktionen umfassten, sowie als Salze vorlagen.

### Beispiel 2

Es wurden 50 Teile Fettsäuregemisch aus Beispiel 1 in ein Reaktionsgefäß überführt und 50 Teile Diethylenglykol zugesetzt. Das Gemisch wurde auf 200 °C erhitzt und Reaktionswasser abdestilliert. Bei 200 °C wurden 0,1 Teile Tetrabutyltitanat zugeführt. Es wurde weiter auf 230 °C erhitzt und hierauf folgend der Druck auf 200 mbar verringert, um die Veresterungsreaktion zu vervollständigen.

Das so gewonnene bräunlich klare Esterpolyol hatte eine Viskosität von 190 mPas/50°C und eine Hydroxylzahl von 505 mg KOH/g.

### Herstellung Polyurethan-Hartschäume

### Beispiel 3

Das in Beispiel 2 erhaltene Esterpolyol wurde mit Additiven formuliert. Hierfür wurden 95 Teile Polyol, 1 Teil Stabilisator DC 193, 0,7 Teil Stabilisator LV 33, 0,3 Teile NP 10 und 3 Teile Wasser gemischt. Anschließend wurde das erhaltene Gemisch mit 170 Teilen Desmodur 44V10 zu einem Polyurethan-Hartschaum umgesetzt.

Der erhaltene Hartschaum hatte eine Dichte von 95 kg/m³.

### Vergleichsbeispiel 1

Es wurde gemäß Beispiel 3 das kommerziell erhältliche Vergleichspolyol Baymer VP PU 25HB55 (95 Teile als Gemisch mit 1 Teil DC 193, 0,7 Teilen LV 33, 0,3 Teilen NP 10 und 3 Teilen Wasser) mit 150 Teilen Desmodur 44V20 zu einem Hartschaum umgesetzt.

Der erhaltene Hartschaum hatte eine Dichte von 96 kg/m³.

Die Ergebnisse der Druckfestigkeits- und Biegefestigkeitsprüfungen sind in Tabelle 1 gezeigt.

**Tabelle 1: Ergebnisse der Druck- und Biegefestigkeitsprüfung.**

| | **Druckfestigkeit (MPa)** | **Biegefestigkeit (MPa)** |
|---|---|---|
| Beispiel 3 | 0,88 | 1,36 |
| Vergleichsbeispiel 1 | 1,01 | 1,17 |

Der erfindungsgemäße Hartschaum aus Beispiel 3 weist eine höhere Biegefestigkeit auf als der Hartschaum aus Vergleichsbeispiel 1, der mit einem marktüblichen Polyol hergestellt wurde. Die Druckfestigkeit des erfindungsgemäßen Hartschaums ist leicht verringert.

Die guten mechanischen Eigenschaften, insbesondere die erhöhte Biegefestigkeit sind überraschend, da es sich bei dem Polyol aus Beispiel 3 um ein Gemisch verschiedener Esterpolyole handelt. Es zeigt sich, dass der Einsatz von erfindungsgemäßen Esterpolyolen in hohen Konzentrationen bei gleichbleibenden und zum Teil verbesserten Eigenschaften möglich ist.

Der Einsatz von Esterpolyolgemischen aus natürlichen Quellen wie Birkenrinde in der Polyurethanhartschaumstoffherstellung führt zu Produkten mit vorteilhaften Eigenschaften. Hierdurch wird eine neuartige Quelle von natürlich vorkommenden Rohstoffen eröffnet, die für die industrielle Nutzung geeignet sind und zugleich Ressourcen schonen, und die zusätzlich noch zu einer Senkung der Herstellungskosten von PU-Schäumen führen.

## Patentansprüche

1. Verfahren zur Herstellung einer Mischung umfassend mindestens eine Verbindung (I) mit mindestens zwei Hydroxy- und/oder Aminogruppen, wobei das Verfahren die folgenden Schritte umfasst:
a) Bereitstellen von mindestens einem Biomasse-Material, bevorzugt Schalen und/oder Rinde, insbesondere Borke und/oder Kork,
b) Behandeln des mindestens einen Biomasse-Materials aus Schritt a) mit mindestens einem ersten organischen Lösungsmittel bei erhöhter Temperatur, nämlich > 20°C, bevorzugt bei 30-200°C,
c) Abtrennen des Feststoffs aus der nach Schritt b) erhaltenen Mischung,
d) Behandeln des nach Schritt c) erhaltenen Feststoffs mit mindestens einem zweiten Lösungsmittel unter alkalischen Bedingungen bei erhöhter Temperatur, nämlich > 20°C, bevorzugt bei 30-200°C,
e) Abtrennen der flüssigen Phase aus der nach Schritt d) erhaltenen Mischung,
f) zumindest teilweises Entfernen des mindestens einen zweiten Lösungsmittels aus der nach Schritt e) erhaltenen flüssigen Phase,
g) Umsetzen der nach Schritt f) erhaltenen Mischung mit mindestens einer Verbindung (II) mit mindestens zwei Hydroxy- und/oder Aminogruppen.

2. Verfahren gemäß Anspruch 1, wobei Schritt b) bei einer Temperatur von 30-200 °C, bevorzugt 50-150 °C, stärker bevorzugt 60-120 °C, insbesondere für 1-300 h, stärker bevorzugt 20-400 h, noch stärker bevorzugt 60-300 h, noch stärker bevorzugt 90-180 h, insbesondere bei vermindertem Druck, bevorzugt bei 150-800 mbar, stärker bevorzugt 150-500 mbar, noch stärker bevorzugt 200-400 mbar durchgeführt wird.

3. Verfahren gemäß einem der vorangehenden Ansprüche, wobei das erste organische Lösungsmittel ausgewählt ist aus Aceton, Dichlormethan, Benzol oder einer Mischung davon und/oder das zweite Lösungsmittel ausgewählt ist aus Isopropanol, Ethanol, Wasser oder einer Mischung davon.

4. Verfahren gemäß einem der vorangehenden Ansprüche, wobei das Abtrennen in Schritt c) Filtration, bevorzugt Heißfiltration, Zentrifugation und/oder Dekantation umfasst.

5. Verfahren gemäß einem der vorangehenden Ansprüche, wobei der pH-Wert in Schritt d) > 7,0 -14,0, und bevorzugt > 8,0 - 14,0 beträgt und wobei Schritt d) insbesondere in Gegenwart eines Hydroxids, bevorzugt NaOH und/oder KOH, eines Carbonats, eines Hydrogencarbonats oder Mischungen davon durchgeführt wird.

6. Verfahren gemäß einem der vorangehenden Ansprüche, wobei Schritt d) bei einer Temperatur von 30-200 °C, bevorzugt 50-150 °C, stärker bevorzugt 60-120 °C, insbesondere für 1-1000 min, stärker bevorzugt 20-500 min, noch stärker bevorzugt 60-300 min, insbesondere 90-180 min, durchgeführt wird.

7. Verfahren gemäß einem der vorangehenden Ansprüche, wobei in Schritt d) C₁₂-C₂₆ Fettsäuren und Derivate davon aus dem Feststoff extrahiert werden.

8. Verfahren gemäß einem der vorangehenden Ansprüche, wobei das Abtrennen in Schritt e) Filtration, bevorzugt Heißfiltration, Zentrifugation und/oder Dekantation umfasst.

9. Verfahren gemäß einem der vorangehenden Ansprüche, wobei in Schritt g) die mindestens eine Verbindung (II) mit mindestens zwei Hydroxy- und/oder Aminogruppen ausgewählt ist aus der Gruppe bestehend aus einem Polyol, Aminopolyol, Polyamin, Polyaminoalkohol, Aminoalkohol, Polyaminopolyol oder einer Mischung, insbesondere Diol, Triol, Diamin, Triamin, Aminoalkohol oder einer Mischung davon, bevorzugt aus Diethylenglykol, Dipropylenglykol, Ethanolamin, Ethylendiamin, Propandiamin, Butandiol, 1,2-Butandiamin, 1,3-Butandiamin, oder einer Mischung davon, insbesondere Diethylenglykol.

10. Verfahren gemäß einem der vorangehenden Ansprüche, wobei Schritt g) bei erhöhter Temperatur, nämlich > 20 °C, bevorzugt 50-400 °C, stärker bevorzugt 150-300 °C, noch stärker bevorzugt 200-300 °C, insbesondere für 30-2400 min, stärker bevorzugt 12-1200 min, noch stärker bevorzugt 180-600 min, insbesondere in Gegenwart eines Katalysators, bevorzugt eines metallorganischen und/oder basischen Katalysators, stärker bevorzugt Titantetrabutanolat, Zinnoctoat, Kaliumacetat, Kaliumhydroxid, Manganacetat, Dibutylzinndilaurat oder einer Kombination davon, bevorzugt Titantetrabutanolat durchgeführt wird.

11. Verfahren gemäß einem der vorangehenden Ansprüche, wobei nach Schritt f) und vor Schritt g) ein weiterer Schritt f1) erfolgt:
f1) zumindest teilweises Umsetzen der nach Schritt f) erhaltenen, Epoxygruppen und/oder ungesättigte C-C Bindungen enthaltenden Mischung in einer Oxidationsreaktion, insbesondere umfassend Ozonolyse oder Wasserstoffperoxid-vermittelte Oxidation, und/oder in einer Additionsreaktion, insbesondere umfassend eine nukleophile Addition oder eine Cycloaddition, stärker bevorzugt eine Diels-Alder-Reaktion, wobei die Additionsreaktion insbesondere Umsetzen mit einem ungesättigten Säureanhydrid, bevorzugt Maleinsäureanhydrid, einem Mono- oder Polyamin, einem Mono- oder Polyol oder einer Mischung davon umfasst.

12. Mischung umfassend mindestens eine Verbindung (I) mit mindestens zwei Hydroxy- und/oder Aminogruppen, erhältlich durch ein Verfahren nach einem der Ansprüche 1-11.

13. Mischung nach Anspruch 12, umfassend mindestens eine Verbindung A, B, C und/oder D mit jeweils mindestens zwei Hydroxy- und/oder Aminogruppen, mit 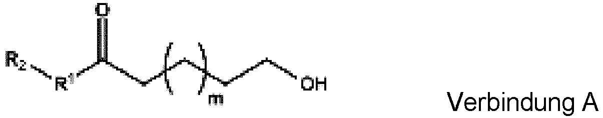
wobei m = 8 - 22 ist; 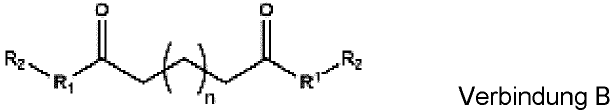
wobei n = 8 - 22 ist; 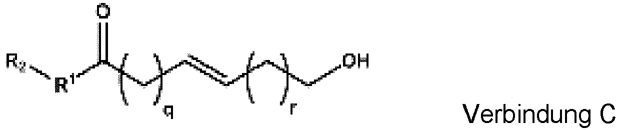
wobei q = 4 - 11 und r = 4 - 11 ist; und/oder 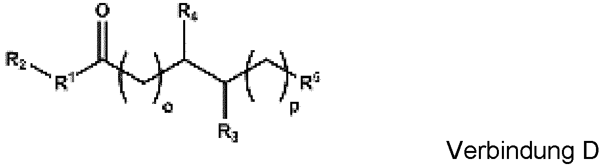
wobei
o = 4 - 11 und p = 4 - 11 ist,
R¹ = O oder NH ist,
R² ausgewählt ist aus der Gruppe bestehend aus einem Monoalkohol, Polyol, Monoamin, Polyamin, Polyaminoalkohol, Aminoalkohol, Aminopolyol, Polyaminopolyol oder einer Mischung davon, insbesondere aus einem Monoalkohol, Diol, Monoamin, Diamin, Aminoalkohol oder einer Mischung davon,
R³ ausgewählt ist aus OH, O-R² oder NH-R²,
R⁴ ausgewählt ist aus OH, O-R² oder NH-R²,
R³ = OH ist, wenn R⁴ = O-R² oder NH-R² ist,
R⁴ = OH ist, wenn R³ = O-R² oder NH-R² ist, und
R⁵ ausgewählt ist aus -COR¹R² und CH₂OH,
wobei der Anteil der Verbindungen A, B, C und/oder D an der Gesamtmischung insbesondere mindestens 20 Gew.% beträgt.

14. Mischung gemäß einem der Ansprüche 12-13, wobei die Mischung eine dynamische Viskosität, bestimmt gemäß DIN 53019, von 1-250.000 mPas/50°C, bevorzugt 1-100.000 mPas/50°C, stärker bevorzugt 1-10.000 mPas/50°C und noch stärker bevorzugt 5-10.000 mPas/50°C und/oder eine Hydroxylzahl, bestimmt gemäß ASTM E 1899-08,von 1-1000 mg KOH/g, bevorzugt 5-800 mg KOH/g, stärker bevorzugt 50-700 mg KOH/g aufweist.

15. Verwendung der Mischung nach einem der Ansprüche 12-14 zur Herstellung eines Polymers, insbesondere eines Polymerschaums, insbesondere zur Herstellung eines Polyurethans, Polyisocyanurats, Polyharnstoffs, Polyesters, Polyamids, Polycarbonats, Polyethers oder einer Kombination hieraus und bevorzugt eines Polyurethans, insbesondere eines Polyurethanschaums.

16. Polyurethan, umfassend eine Mischung nach einem der Ansprüche 12-14 als ein Polymerbaustein.

## Claims

1. Method for producing a mixture comprising at least one compound (I) having at least two hydroxy and/or amino groups, wherein the method comprises the following steps:
a) providing at least one biomass material, preferably husks and/or bark, in particular tree bark and/or cork,
b) treating the at least one biomass material from step a) with at least one first organic solvent at elevated temperature, namely > 20°C, preferably at 30-200°C,
c) separating off the solid from the mixture obtained after step b),
d) treating the solid obtained after step c) with at least one second solvent under basic conditions at elevated temperature, namely > 20°C, preferably at 30-200°C,
e) separating off the liquid phase from the mixture obtained after step d),
f) at least partially removing the at least one second solvent from the liquid phase obtained after step e),
g) reacting the mixture obtained after step f) with at least one compound (II) having at least two hydroxy and/or amino groups.

2. Method according to claim 1, wherein step b) is carried out at a temperature of 30-200°C, preferably 50-150°C, more preferably 60-120°C, in particular for 1-300 h, more preferably 20-400 h, even more preferably 60-300 h, even more preferably 90-180 h, in particular at reduced pressure, preferably at 150-800 mbar, more preferably 150-500 mbar, even more preferably 200-400 mbar.

3. Method according to either of the preceding claims, wherein the first organic solvent is selected from acetone, dichloromethane, benzene or a mixture thereof, and/or the second solvent is selected from isopropanol, ethanol, water or a mixture thereof.

4. Method according to one of the preceding claims, wherein the separation in step c) comprises filtration, preferably hot filtration, centrifugation and/or decantation.

5. Method according to one of the preceding claims, wherein the pH in step d) is > 7.0 - 14.0, and preferably > 8.0 - 14.0, and wherein step d) is carried out in particular in the presence of a hydroxide, preferably NaOH and/or KOH, a carbonate, a hydrogen carbonate or mixtures thereof.

6. Method according to one of the preceding claims, wherein step d) is carried out at a temperature of 30-200°C, preferably 50-150°C, more preferably 60-120°C, in particular for 1-1000 min, more preferably 20-500 min, even more preferably 60-300 min, in particular 90-180 min.

7. Method according to one of the preceding claims, wherein, in step d), C₁₂-C₂₆ fatty acids and derivatives thereof are extracted from the solid.

8. Method according to one of the preceding claims, wherein the separation in step e) comprises filtration, preferably hot filtration, centrifugation and/or decantation.

9. Method according to one of the preceding claims, wherein, in step g), the at least one compound (II) having at least two hydroxy and/or amino groups is selected from the group consisting of a polyol, aminopolyol, polyamine, polyamino alcohol, amino alcohol, polyaminopolyol or a mixture, in particular diol, triol, diamine, triamine, amino alcohol or a mixture thereof, preferably from diethylene glycol, dipropylene glycol, ethanolamine, ethylenediamine, propanediamine, butanediol, 1,2-butanediamine, 1,3-butanediamine or a mixture thereof, in particular diethylene glycol.

10. Method according to one of the preceding claims, wherein step g) is carried out at elevated temperature, namely > 20 °C, preferably 50-400°C, more preferably 150-300°C, even more preferably 200-300°C, in particular for 30-2400 min, more preferably 12-1200 min, even more preferably 180-600 min, in particular in the presence of a catalyst, preferably an organometallic and/or basic catalyst, more preferably titanium tetrabutoxide, tin octoate, potassium acetate, potassium hydroxide, manganese acetate, dibutyltin dilaurate or a combination thereof, preferably titanium tetrabutoxide.

11. Method according to one of the preceding claims, wherein, after step f) and before step g), a further step f1) is performed:
f1) at least partially reacting the mixture containing epoxy groups and/or unsaturated C-C compounds obtained after step f) in an oxidation reaction, in particular comprising ozonolysis or hydrogen peroxide-mediated oxidation, and/or in an addition reaction, in particular comprising nucleophilic addition or cycloaddition, more preferably a Diels-Alder reaction, wherein the addition reaction in particular comprises reaction with an unsaturated acid anhydride, preferably maleic anhydride, with a monoamine or polyamine, a monool or polyol, or a mixture thereof.

12. Mixture comprising at least one compound (I) having at least two hydroxy and/or amino groups, obtainable by means of a method according to one of claims 1-11.

13. Mixture according to claim 12, comprising at least one compound A, B, C and/or D, each having at least two hydroxy and/or amino groups, with 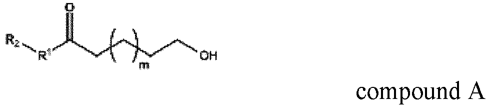
wherein m = 8 - 22; 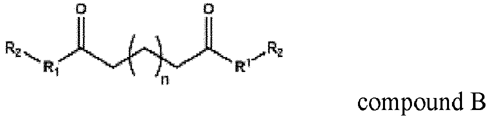
wherein n = 8 - 22; 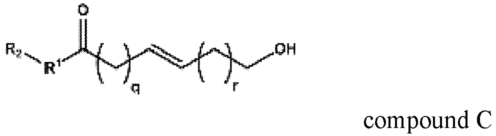
wherein q = 4 - 11 and r = 4 - 11; and/or 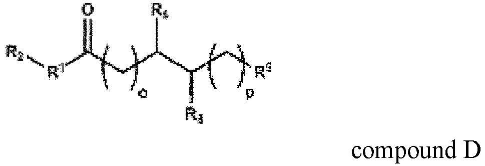
wherein
o = 4 - 11 and p = 4 - 11,
R¹ = O or NH,
R² is selected from the group consisting of a monoalcohol, polyol, monoamine, polyamine, polyamino alcohol, amino alcohol, aminopolyol, polyaminopolyol or a mixture thereof, in particular from a monoalcohol, diol, monoamine, diamine, amino alcohol or a mixture thereof,
R³ is selected from OH, O-R² or NH-R²,
R⁴ is selected from OH, O-R² or NH-R²,
R³ = OH, when R⁴ = O-R² or NH-R²,
R⁴ = OH, when R³ = O-R² or NH-R², and
R⁵ is selected from -COR¹R² and CH₂OH,
wherein the proportion of the compounds A, B, C and/or D in the total mixture is in particular at least 20 wt%.

14. Mixture according to either of claims 12 and 13, wherein the mixture has a dynamic viscosity, determined according to DIN 53019, of 1-250,000 mPas/50°C, preferably 1-100,000 mPas/50°C, more preferably 1-10,000 mPas/50°C and even more preferably 5-10,000 mPas/50°C and/or a hydroxyl value, determined according to ASTM E 1899-08, of 1-1000 mg KOH/g, preferably 5-800 mg KOH/g, more preferably 50-700 mg KOH/g.

15. Use of the mixture according to one of claims 12-14 for the production of a polymer, in particular a polymer foam, in particular for the production of a polyurethane, polyisocyanurate, polyurea, polyester, polyamide, polycarbonate, polyether or a combination thereof, and preferably of a polyurethane, in particular a polyurethane foam.

16. Polyurethane comprising a mixture according to one of claims 12-14 as a polymer building block.

## Revendications

1. Procédé de préparation d'un mélange comprenant au moins un composé (I) ayant au moins deux groupes hydroxy et/ou amino, ledit procédé comprenant les étapes suivantes consistant à :
a) fournir au moins un matériau de biomasse, de préférence des coquilles et/ou des écorces, en particulier de l'écorce externe et/ou du liège,
b) traiter ledit au moins un matériau de biomasse de l'étape a) avec au moins un premier solvant organique à une température élevée, à savoir à une température > 20 °C, de préférence de 30 à 200 °C,
c) séparer la matière solide du mélange obtenu à l'étape b),
d) traiter la matière solide obtenue à l'étape c) avec au moins un deuxième solvant dans des conditions alcalines à une température élevée, à savoir à une température > 20 °C, de préférence de 30 à 200 °C,
e) séparer la phase liquide du mélange obtenu à l'étape d),
f) éliminer au moins partiellement ledit au moins un deuxième solvant de la phase liquide obtenue à l'étape e),
g) faire réagir le mélange obtenu à l'étape f) avec au moins un composé (II) ayant au moins deux groupes hydroxy et/ou amino.

2. Procédé selon la revendication 1,
dans lequel l'étape b) est réalisée à une température de 30 à 200 °C, de préférence de 50 à 150 °C, plus préférentiellement de 60 à 120 °C, en particulier pendant 1 à 300 h, de préférence pendant 20 à 400 h, plus préférentiellement pendant 60 à 300 h, encore plus préférentiellement pendant 90 à 180 h, en particulier à une pression réduite, de préférence à une pression de 150 à 800 mbars, plus préférentiellement de 150 à 500 mbars, encore plus préférentiellement de 200 à 400 mbars.

3. Procédé selon l'une des revendications précédentes,
dans lequel le premier solvant organique est choisi parmi l'acétone, le dichlorométhane, le benzène ou un mélange de ceux-ci, et/ou le deuxième solvant est choisi parmi l'isopropanol, l'éthanol, l'eau ou un mélange de ceux-ci.

4. Procédé selon l'une des revendications précédentes,
dans lequel la séparation à l'étape c) comprend une filtration, de préférence une filtration à chaud, une centrifugation et/ou une décantation.

5. Procédé selon l'une des revendications précédentes,
dans lequel le pH à l'étape d) est > 7,0 à 14,0, et de préférence > 8,0 à 14,0, et l'étape d) est réalisée en particulier en présence d'un hydroxyde, de préférence NaOH et/ou KOH, d'un carbonate, d'un hydrogénocarbonate ou de leurs mélanges.

6. Procédé selon l'une des revendications précédentes,
dans lequel l'étape d) est réalisée à une température de 30 à 200°C, de préférence de 50 à 150°C, plus préférentiellement de 60 à 120°C, en particulier pendant 1 à 1000 min, plus préférentiellement pendant 20 à 500 min, encore plus préférentiellement pendant 60 à 300 min, en particulier pendant 90 à 180 min.

7. Procédé selon l'une des revendications précédentes,
dans lequel, à l'étape d), les acides gras en C₁₂-C₂₆ et leurs dérivés sont extraits de la matière solide.

8. Procédé selon l'une des revendications précédentes,
dans lequel la séparation à l'étape e) comprend une filtration, de préférence une filtration à chaud, une centrifugation et/ou une décantation.

9. Procédé selon l'une des revendications précédentes,
dans lequel, à l'étape g), ledit au moins un composé (II) ayant au moins deux groupes hydroxy et/ou amino est choisi dans le groupe constitué par un polyol, un aminopolyol, une polyamine, un polyaminoalcool, un aminoalcool, un polyaminopolyol ou un mélange de ceux-ci, en particulier un diol, un triol, une diamine, une triamine, un aminoalcool ou un mélange de ceux-ci, de préférence le diéthylèneglycol, le dipropylèneglycol, l'éthanolamine, l'éthylènediamine, la propanediamine, le butanediol, la 1,2-butanediamine, la 1,3-butanediamine, ou un mélange de ceux-ci, en particulier le diéthylèneglycol.

10. Procédé selon l'une des revendications précédentes,
dans lequel l'étape g) est réalisée à une température élevée, à savoir à une température > 20 °C, de préférence de 50 à 400 °C, plus préférentiellement de 150 à 300 °C, encore plus préférentiellement de 200 à 300 °C, en particulier pendant 30 à 2400 min, plus préférentiellement pendant 12 à 1200 min, encore plus préférentiellement pendant 180 à 600 min, en particulier en présence d'un catalyseur, de préférence un catalyseur organométallique et/ou basique, plus préférentiellement le tétrabutanolate de titane, l'octoate d'étain, l'acétate de potassium, l'hydroxyde de potassium, l'acétate de manganèse, le dilaurate de dibutylétain ou une combinaison de ceux-ci, de préférence le tétrabutanolate de titane.

11. Procédé selon l'une des revendications précédentes,
dans lequel, après l'étape f) et avant l'étape g), on effectue une étape supplémentaire f1) :
f1) faire réagir au moins partiellement le mélange, obtenu à l'étape f), contenant des groupes époxy et/ou des liaisons C-C insaturées, dans une réaction d'oxydation, comprenant en particulier une ozonolyse ou une oxydation médiée par le peroxyde d'hydrogène, et/ou dans une réaction d'addition, comprenant en particulier une addition nucléophile ou une cycloaddition, plus préférablement une réaction de Diels-Alder, la réaction d'addition comprenant en particulier la réaction avec un anhydride d'acide insaturé, de préférence l'anhydride maléique, une mono- ou polyamine, un mono- ou polyol, ou un mélange de ceux-ci.

12. Mélange comprenant au moins un composé (I) ayant au moins deux groupes hydroxy et/ou amino, pouvant être obtenu par un procédé selon l'une des revendications 1 à 11.

13. Mélange selon la revendication 12, comprenant au moins un composé A, B, C et/ou D ayant chacun au moins deux groupes hydroxy et/ou amino, avec 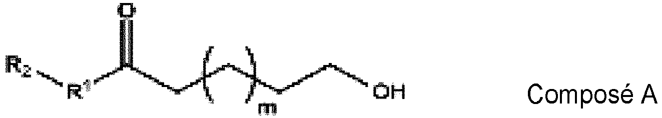
où m = 8 à 22 ; 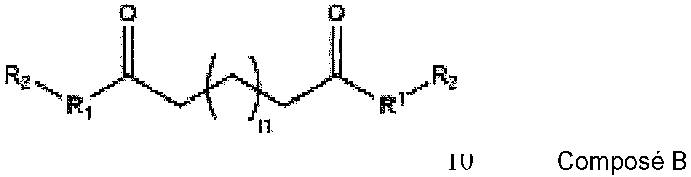
où n = 8 à 22 ; 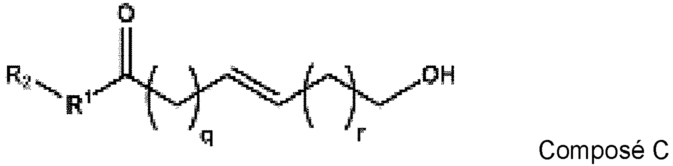
où q = 4 à 11 et r = 4 à 11 ; et/ou 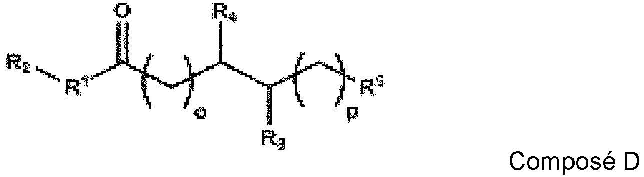
où o = 4 à 11 et p = 4 à 11,
R¹ = O ou NH,
R² est choisi dans le groupe constitué par un monoalcool, un polyol, une monoamine, une polyamine, un polyaminoalcool, un aminoalcool, un aminopolyol, un polyaminopolyol ou un mélange de ceux-ci, en particulier un monoalcool, un diol, une monoamine, une diamine, un aminoalcool ou un mélange de ceux-ci,
R³ est choisi parmi OH, O-R² ou NH-R²,
R⁴ est choisi parmi OH, O-R² ou NH-R²,
R³ = OH si R⁴ = O-R² ou NH-R²,
R⁴ = OH si R³ = O-R² ou NH-R², et
R⁵ est choisi parmi -COR¹R² et CH₂OH,
la proportion des composés A, B, C et/ou D par rapport au mélange total étant d'au moins 20 % en poids.

14. Mélange selon l'une des revendications 12 à 13,
dans lequel le mélange présente une viscosité dynamique, déterminée selon DIN 53019, de 1 à 250 000 mPas/50 °C, de préférence de 1 à 100 000 mPas/50 °C, plus préférentiellement de 1 à 10 000 mPas/50 °C et encore plus préférentiellement de 5 à 10 000 mPas/50 °C, et/ou un indice d'hydroxyle, déterminé selon ASTM E 1899-08, de 1 à 1 000 mg KOH/g, de préférence de 5 à 800 mg KOH/g, plus préférentiellement de 50 à 700 mg KOH/g.

15. Utilisation du mélange selon l'une des revendications 12 à 14 pour la préparation d'un polymère, en particulier d'une mousse de polymère, en particulier pour la préparation d'un polyuréthane, d'un polyisocyanurate, d'une polyurée, d'un polyester, d'un polyamide, d'un polycarbonate, d'un polyéther ou une d'combinaison de ceux-ci et de préférence d'un polyuréthane, en particulier d'une mousse de polyuréthane.

16. Polyuréthane comprenant un mélange selon l'une des revendications 12 à 14 en tant que composant polymère.
